Europäisches Patentamt

European Patent Office

Office européen des brevets

(11) Publication number: **0 006 406**
A1

# EUROPEAN PATENT APPLICATION

(21) Application number: 78100309.0

(22) Date of filing: **05.07.78**

(51) Int. Cl.³: **A 61 L 15/00**, A 61 F 13/00 // A61B19/00

(43) Date of publication of application: **09.01.80** Bulletin 80/1

(84) Designated Contracting States: **DE FR GB SE**

(71) Applicant: **FUJI SYSTEMS CORPORATION, 11-1, Ebisu 1-chome Shibuya-Ku, Tokyo (JP)**

(72) Inventor: **Kawaguchi, Nobuhisa, No.7-29,Sasame-Machi, Kamakura-Shi Kanagawa-ken (JP)** Inventor: **Inaba, Yutaka, No.30-12, Yushima 2-chome, Bunkyo-ko Tokyo (JP)**

(74) Representative: **Kern, Ralf, Dipl.-Ing. et al, Henkel, Kern, Feiler & Hänzel Patentanwälte Möhlstrasse 37, D-8000 München 80 (DE)**

(54) Cover sheet for brain and spinal cord in neurosurgical operation.

(57) The invention provides a cover for use in neuro-surgical operations on brain and spinal cord, which consists of a porous sheet (1) of a hydrophilic polymer substance having a substantially continuous cellular structure and being non-reactive to living body tissues. The sheet (1) may have a thread (2) attached thereto.

ACTORUM AG

0006406

Möhlstraße 37
D-8000 München 80

Tel.: 089/982085-87
Telex: 0529802 hnkl d
Telegramme: ellipsoid

- 1 -

## Cover sheet for brain and spinal cord in neurosurgical operation

The present invention relates to a cover which is to be placed on the brain and spinal cord, for the purpose of protecting them against drying and damages during the operation.

Neurosurgical operations on the brain and spinal cord involve an extreme danger, and an inadvertent damage on the essential part thereof is on occasion fatal or may be attended with serious postoperative complication subsequent to the operation. The circulation of blood in the brain is so vigorous that even an injury inflicted on a small vessel would cause an intense bleeding. Accordingly, in order to protect those parts of the neurosurgical operation field which need no operative intervention, to prevent them from getting dry, and to minimize mechanical damages thereon during operation, it is necessary to provide a cover to be placed on the brain and spinal cord. As a cover for this purpose in the prior art, there is used one prepared by cutting gauze, absorbent cotton or nonwoven fabric after the shape of the operation field, sterilizing the thus cut material and thereafter wetting it with a physiological saline solution for softening. However, in the case of an operation requiring much time, a cover of this kind would partially get dry, causing such troubles that a portion of the brain

- 2 -                    0006406

tissue peels off along with the cover at the time of the removal thereof, or a part of fibers of the cover is inadvertently left behind as a foreign substance in the brain tissue.

Besides, the fiber material is apt to be finely splitted and to get fluffy easily, so the visual field under the operating microscope is not infrequently obstructed.

The brain tissue is very soft to the touch like pudding and is apt to be injured. Accordingly, a cover sheet for the purpose of covering the surface of the operation field during the neurosurgical operation so as to protect the brain tissue against damages should meet such requirements as follows:

1. It should touch the brain tissue very softly.
2. Since the operation field is exposed to an illuminant and the brain tissue tends to get dry, it should have a sufficient water-holding capacity.
3. There should not be caused falling-off of any substance including fibers at the time of removing the cover sheet from the surface of the brain tissue.
4. It should be water-insoluble, essentially non-reactive to body tissues as well as fluids, and harmless to the patient.
5. It should permit to conduct the suction of fluid such as saline solution, cerebrospinal fluid and/or blood from above the cover sheet.
6. It should be uniformly thin and not fluffy so as not to obstruct the view at the time of conducting operation under the operating microscope.

The present invention has as its object to provide a cover for use in neurosurgical operation on brain and spinal cord which is capable of sufficiently meeting the aforementioned requirements. The invention as characterized in the enclosed claims solves this object by providing a cover which is non-reactive to body tissues, said cover being formed of a porous sheet of a hydrophilic polymer substance which is water-absorbent, hydrophilic, water-insoluble, chemical resistant and pliable and has a substantially continuous cellular structure.

Below, the invention is explained in greater detail by referring to the accompanying drawing illustrating exemplary embodiments and wherein:

Fig. 1 is a perspective view of a square cover according to the invention;

Fig. 2 is a perspective view of a circular cover; and

Figs. 3, 4 and 5 are respectively a perspective view of the same square cover with a colored thread attached in various manners.

The cover 1 is made of a porous sheet of a hydrophilic polymer substance having a substantially continuous cellular structure. The thickness of the cover 1 is preferably in the range of from about 0.5 mm to about 3 mm, and said thickness is required to be uniform. As to the shape of the cover 1, it can be of optional shape in addition to square, rectangular, circle and semicircle, that is, it can be selected in conformity with the shape of the operation field. The dimensions of the cover 1 depend on the size of the operation field; in the case where it is square, the length of sides thereof is usually in the range of from about 5 mm to about 9 cm, and a plurality of such square members are arranged side by side in a mosaic fashion leaving no space in the surroundings of the exposed operation field, so as to serve as a cover.

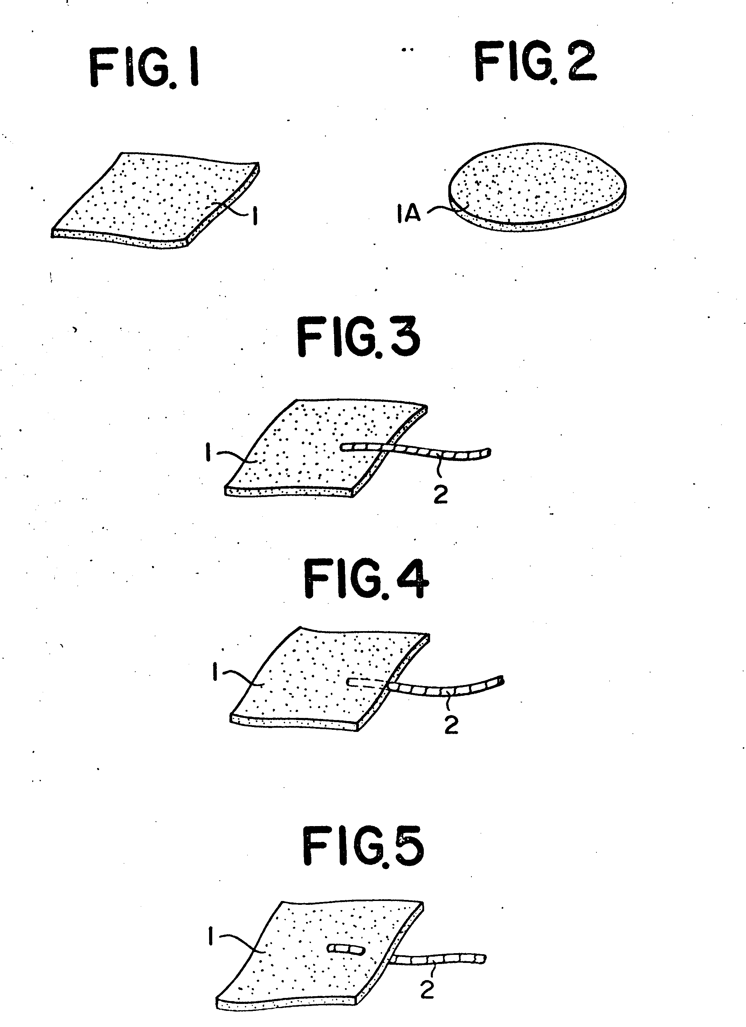

The cover 1 must be taken off after using it in neurosurgical operations on the brain and spinal cord, and therefore, especially when it is of a small size, it is preferably provided with a thread 2 having a particularly conspicuous color, such as green or black, lest it should be mislaid in the operation field. Fig. 3 illustrates a mode wherein one end of the thread 2 is fixed to the surface of the cover 1, Fig. 4 illustrates another mode wherein one end of the thread 2 is fixed to the inner part of the cover 1, and Fig. 5 illustrates still another mode wherein one end of the thread 2 penetrating from one side to the other side of the cover 1 is fixed to the surface of the cover. As the material for the thread 2, Dacron, nylon, etc., are useful, and the fixing of the thread 2 onto the cover 1 can be effected by means of an adhesive such as ethylene-vinyl acetate copolymer, but it is preferable to adopt welding. Further, in order to make sure whether the cover is mislaid in the operation field prior to suturing the operated part, the thread is impregnated with an X-ray contrast medium such as barium sulfate, tantalum powder, silver powder, etc., to the extent of about 20 to about 50% by weight to serve for preventing the risk of mislaying the cover in the operation field.

As the porous sheet of a hydrophilic polymer substance having a substantially continuous cellular structure to be employed in the present invention, any sheet is useful as far as it is water-absorbent, hydrophilic, water-insoluble, resistant to chemicals, pliable, non-reactive to the body tissue, and harmless to the patient, and especially polyvinylformal is apposite. A porous sheet of polyvinylformal (60 to 65 mole percent formalized) can be simply obtained from a sheet of polyvinylformal mixed with water-soluble or solvent-soluble fine powder by causing said fine powder to effuse. It also will do to employ a sheet obtained by slicing, in a thickness required, a sponge formed by adding a foaming agent at the time of formalization.

In the aforementioned properties required for the porous sheet, the following is particularly important. That is, water-absorbent property can be expressed by weight (Wa), the maximum water content, and in the equation

$$Wa = \frac{(\text{weight when sheet absorbed water sufficiently}) - (\text{dry weight})}{\text{dry weight}} \times 100,$$

Wa is desirably in the range of from 800 to 1900%. The porous property can be expressed by porosity (Pr), and in the equation

$$Pr = \frac{(\text{apparent volume}) - (\text{actual volume of material})}{\text{apparent volume}} \times 100,$$

Pr is desirably in the range of from 85 to 95%. The pliability can be expressed by compressive stress (Mc, and it is desirable that the value measured according to German Industrial Standard DIN 53577, wherein the load per 1 $cm^2$ necessary for compressing a certain wet thickness of sheet to reduce to 30% is reckoned as the compressive stress thereof, be preferably in the range of from about 10 to 55 $g/cm^2$, in particular, superior results can be effected when said value is in the range from 15 to 30 $g/cm^2$. All of the aforementioned values have been confirmed as a result of tests on the physical stimulus conducted by applying the cover of the present invention to the body tissues.

Since the present cover is excellent in water absorbency as well as water retaining capability, it is practically free from becoming dry while in use for operations. Besides, because of its of spongy structure, not knitted or woven structure, unlike the conventional covers, it is absolutely free from falling-off of fibers. Moreover, it is absolutely insoluble in water in spite of its hydrophilic property, free from degeneration due to medical stuffs and harmless to body tissues. Consequently, it is very useful as a cover of this sort from the viewpoint of practical use.

Example:

Three varieties of cellular type polyvinyl formal sponge Specimens A, B and C having the following values in respect of Wa, Pr and Mc, respectively were obtained from the composition of polyvinyl alcohol, hydrochloric acid, formalin and starch, wherein the polyvinyl alcohol has a mean degree of polymerization of about 500, the hydrochloric acid is used as a catalyst and the starch is used as a pore-forming agent.

| Specimen No. | Rate of formalization (mole %) | Wa (%) | Pr (%) | Mc ($g/cm^2$) |
|---|---|---|---|---|
| A | about 50 | 1900 | 90 | 12 |
| B | about 60 | 1300 | 90 | 27 |
| C | about 75 | 800 | 90 | 51 |

Each specimen was sliced into a sheet having a thickness of 1 mm.  The sliced sheet was washed in hot water, dried under dustfree conditions and then cut into 1 cm x 2 cm small pieces with scissors.  One end of a 20 cm long and 0.203 mm thick polyester stitching thread for use in operations was fixed onto the surface of said piece by the aid of an ethylene-vinyl acetate copolymer to thereby obtain a cover sheet as illustrated in Fig. 1.  This cover sheet was sterilized with ethylene oxide gas and then stored in a sterilized physiological saline solution.  Thus prepared cover sheet specimens were tested in 3 hours' dog craniotomy.  From the obtained results it was observed that Specimen B is most superior in pliability, strength and absorbency; Specimen A is inferior in absorbency to Specimen B; and Specimen C is inferior in hydrophile property and pliability  to Specimen B.

- 1 -                                    0006406

Patent Claims

1.  A cover for use in neurosurgical  operation on the
    cerebrospinal medulla, characterized in that said
    cover is non-reactive to body tissues and is formed
    of a porous sheet (1) of a hydrophilic polymer substance being water-absorbent, hydrophilic, water-
    insoluble, resistant to chemicals and pliable and
    having a substantially continuous cellular structure,
    said porous sheet (1) having a thickness of about 0.5
    to about 3 mm, a water-holding capacity of about 8 to
    about 19 times as much as the dry weight of the sheet,
    porosity of  about 85 to about 95%, and an excellent
    pliability in wet condition.

2.  A cover according to Claim 1, characterized in that
    said porous sheet (1) is provided with a colored thread
    (2) fixed thereto.

3.  A cover according to Claim 2, characterized in that
    said colored thread (2) contains an X-ray contrast medium.

4.  A cover according to Claim 1, characterized in that
    said porous sheet (1) is made of polyvinylformal.

5.  A cover according to Claim 1, characterized in that
    said porous sheet (1) is made of viscose sponge.

0006406

## FIG. 1

1

## FIG. 2

1A

## FIG. 3

1

2

## FIG. 4

1

2

## FIG. 5

1

2

0006406

European Patent Office
EUROPEAN SEARCH REPORT
Application number
EP 78 10 0309

| | DOCUMENTS CONSIDERED TO BE RELEVANT | | CLASSIFICATION OF THE APPLICATION (Int. Cl.²) |
|---|---|---|---|
| Category | Citation of document with indication, where appropriate, of relevant passages | Relevant to claim | |
| X | FR – A – 2 337 028 (ROSENBLATT) <br> * Page 2, lines 28-30; page 5, line 27 to page 13, line 18 * | 1,2,3, 4 | A 61 L 15/00 <br> A 61 F 13/00// <br> A 61 B 19/00 |
| X | GB – A – 786 100 (BARENT) <br> * Page 1, lines 47 to 49; page 2, lines 85 to 100 * | 1,4 | |
| X | GB – A – 1 390 240 (NYLONGE) <br> * Page 1, lines 74 to 86; page 2, lines 23 to 34 * | 1,5 | TECHNICAL FIELDS SEARCHED (Int.Cl.²) <br><br> A 61 B <br> A 61 F <br> A 61 L |
| X | FR – A – 2 125 527 (CODMAN) <br> * Page 2, line 27 to page 4, line 2 * | 1,2,3, 4 | |
| | FR – A – 2 258 188 (SAINT DENIS SOFACO) <br> * Page 1, line 40 to page 2, line 4 * | 5 | |
| | FR – A – 1 568 149 (KANBAR) <br> * Page 2, left-hand column, 6th paragraph; page 3, left-hand column, 3rd paragraph; page 3, right-hand column, point 5 of the abstract * | 1,4 | CATEGORY OF CITED DOCUMENTS <br><br> X: particularly relevant <br> A: technological background <br> O: non-written disclosure <br> P: intermediate document <br> T: theory or principle underlying the invention <br> E: conflicting application <br> D: document cited in the application <br> L: citation for other reasons |
| | The present search report has been drawn up for all claims | | &: member of the same patent family, corresponding document |

| Place of search | Date of completion of the search | Examiner |
|---|---|---|
| The Hague | 20-03-1979 | LEMERCIER |

EPO Form 1503.1   06.78